# EUROPEAN PATENT APPLICATION

(11) **EP 0 562 612 A1**
(43) Date of publication of application: **29.09.1993**
(21) Application number: 93104975.3
(22) Date of filing: 25.03.1993
(51) Int. Cl.: A61L 27/00

(54) **Bioabsorbable blends of a bioabsorbable copolymer and a poly(oxyalkylene)**

(30) Priority: 25.03.1992 US 857616
(71) Applicant: United States Surgical Corporation, Norwalk, Connecticut 06856 (US)
(72) Inventor: Muth, Ross R., Brookfield, CT 06804 (US); Totakura,, Nagabhushanam, Norwalk, CT 06851 (US)
(74) Representative: Marsh, Roy David

(57) **Abstract**

Bioabsorbable compositions useful for coating medical devices include a blend or physical mixture of a poly(oxyalkylene), such as polyethylene oxide, and a bioabsorbable copolymer having soft segments, such as glycolide-trimethylene carbonate copolymer.

## Description

### FIELD OF THE INVENTION

This invention relates generally to novel bioabsorbable compositions. More particularly, this invention relates to new bioabsorbable polymer blends useful for coating medical devices.

### BACKGROUND OF OF THE INVENTION

Bioabsorbable coatings are applied to a wide range of medical devices for a variety of reasons. The nature of the medical device used as the substrate for the coating will normally determine which particular characteristics are desired in the coating. For example, implantable porous prostheses for use as bone or other hard tissue replacements are usually formed from polymeric beads or particles having a biologically compatible, hydrophilic coating. The hydrophilic coating on the particles facilitates infusion of body fluids into the pores of the implant, thereby facilitating the ingrowth of tissue into the pores of the implant. Such prostheses are described in U.S. Patent Nos. 4,728,570; 4,535,485; 4,547,327; and 4,536,158.

As another example, synthetic vascular grafts made from tubes of fabric may be manufactured from yarns coated with a hydrophilic coating. In addition, a surface of the fabric may be coated with an absorbable coating to temporarily render the otherwise porous fabric impervious to blood and/or other body fluids. It is desirable that the fluids-occluding coating exhibit sufficient elasticity to accommodate the alternate elongation and contraction which the tubular fabric prothesis undergoes when implanted in the body. Such tubular fabric protheses are described in U.S. Patent No. 4,990,158.

In certain applications, a medical device may advantageously include a hydrophobic coating. Hydrophobic coatings provide desirable handling characteristics, allowing easy and accurate positioning of the device, better holding of knots, and decreased slipping in the gloved hands of surgeons. Additionally, medical devices with hydrophobic coatings may provide improved tissue retaining properties, for example, where it is desired to hold tissue together.

Copolymers of glycolide and trimethylene carbonate (also referred to as 1,3-dioxan-2-one) have been used to fabricate bioabsorbable medical devices or surgical articles. See for example, U.S. Patent Nos. 4,243,775; 4,300,565; 4,429,080; 4,633,873; and 4,719,917. Additionally, glycolide-trimethylene carbonate (GTMC) polymers have been applied to sutures and other surgical articles as a bioabsorbable coating. See U.S. Patent Nos. 4,705,820 and 4,788,979. GTMC polymers have also been formed into filaments and braided with non-absorbable components (see U.S. Patent No. 4,792,336) and used to coat or encapsulate a woven mesh or other textile structures formed by filaments of non-absorbable polymers (see European Patent Application No. 0334046).

U.S. Patent No. 4,452,973 describes poly(glycolic acid)/poly(oxyalkylene) ABA triblock copolymers useful as absorbable sutures.

### SUMMARY OF THE INVENTION

It has now been found that blends of poly(oxyalklylene) with a bioabsorbable copolymer having soft segments are useful as bioabsorbable compositions, such as, for example bioabsorbable coatings for medical devices. These blends provide coatings which have good elastic, durability and hydrophilic and/or hydrophobic properties as required for a particular end use. The soft segments of the copolymer may be formed by incorporating into the copolymer a monomer selected from the group consisting of trimethylene carbonate, dioxanone, caprolactone, alkylene oxalates, hydroxybutyrates, esteramides, hydroxyvalerates, urethanes, hexamethylene carbonate and mixtures thereof. The blend optionally includes a medico-surgically useful substance, such as, for example, an antithrombogenic substance.

### DESCRIPTION OF PREFERRED EMBODIMENTS

The compositions of the present invention comprise polymer blends containing or physical mixtures of: (a) a bioabsorbable copolymer having soft segments; and (b) poly(oxyalkylene).

Bioabsorbable copolymers suitable for preparing the blends of this invention contain one or more comonomer which provide soft segments. For example, soft segments may be incorporated into polymers of glycolide, lactide or glycolide lactide copolymers by incorporating trimethylene carbonate as a comonomer during polymerization. Other comonomers suitable for generating soft segments include dioxanone, caprolactone, alkylene oxalates, hydroxybutyrates, esteramides, hydroxyvalerates, urethanes, hexamethylene carbonate and mixtures thereof. Trimethylene carbonate, dioxanone and caprolactone are the preferred comonomers. These comonomers may be present in an amount tip to 75 mole percent, and preferably between about 65 mole percent.

The bioabsorbable copolymer used in the present invention may be formed by copolymerizing one or more of the aforementioned soft segment comonomers with one or more monomers known to produce a bioabsorbable polymer, such as, for example, glycolide or lactide. Preferred bioabsorbable copolymer include copolymers of glycolide and trimethylene carbonate.

Glycolide-trimethylene carbonate copolymers employed in particularly useful embodiments of the present invention may contain tip to about 50 mole percent glycolide and up to about 75 mole percent trimethylene carbonate. A preferred copolymer is glycolide/trimethylene carbonate (GTMC) copolymer containing about 35 mole percent glycolide and about 65 mole percent trimethylene carbonate.

Polyoxyalkylenes useful in this invention include those which are hydrophilic in nature, such as, for example, polyethylene oxide, poly(oxyethylene-oxypropylene) copolymers and block copolymers of polyoxyethylene and polyoxypropylene commercially available under the tradename Pluronic. Hydrophobic polyethers may also be used in the present blends. Suitable hydrophobic polyethers include polypropylene oxide, polypentylene oxide and poly-1,4-butane diol.

Polyethylene oxide (PEO) polymers suitable for use in this invention are commercially available in a wide variety of molecular weights. Preferred polyethylene oxide polymers are those having a molecular weight from about 400 to about 20,000. Most preferred is a polyethylene oxide with a molecular weight of 8000. Generally, as the amount of polyethylene oxide in the blend is increased, the hydrophylicity of the blend will increase. The polymer blends may include poly(oxyalkylene) in an amount up to about 50 percent based on the total weight of the blend. Preferably, the blend will contain between about 10 and 20 percent polyalkylene oxide based on the total weight of the blend.

The compositions of the present invention may be formulated to possess a desired set of characteristics depending on the application in which the compositions are to be used. For example, the hydrophobic or hydrophilic nature of the composition may be adjusted by the choice of polyalkylene oxide or mixture of polyalkylene oxides. A balance of hydrophilic and hydrophobic characteristics are achievable by the compositions of the present invention. As another example, the rate at which the composition bioabsorbs may be adjusted by varying the amounts of glycolide and trimethylene carbonate in the copolymer.

Other components may be included in the compositions of this invention such that the coating compositions are employed as a carrier for one or more medico-surgically useful substances, e.g.,those which accelerate or otherwise beneficially modify the healing process when applied to a wound or surgical site. In general, any biologically active material which is soluble in and otherwise compatible with the selected coating composition can be incorporated therein in therapeutically useful amounts. For example, a therapeutic agent may be chosen for its antimicrobial properties, capability for promoting wound repair and/or tissue growth or for specific indications such as thrombolysis. Antimicrobial agents such as broad spectrum antibiotics (gentamycin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or several growth promoting factors can be added to the coating, e.g., fibroblast growth factor, bone growth factor, epidermal growth factor, platelet derived growth factor, macrophage derived growth factor, alveolar derived growth factor, monocyte derived growth factor, magainin, and so forth. Some therapeutic indications are: glycerol with tissue or kidney plasminogen activator to cause thrombolysis, superoxide dismutase to scavenge tissue damaging free radicals, tumor necrosis factor for cancer therapy or colony stimulating factor and interferon, interleukin-2 or other lymphokine to enhance the immune system. Other examples of materials which may be added to the blends of this invention include antithrombogenic agents (such as heparin, hirudin and prostaglandins), pharmacologically active agents, anti-coagulants, osteogenic factors, anti-fungals, immunosuppressive agents, anti-inflammatory agents, preservatives, saccharides, diagnostic agents, antihistamines, hormones, enzymes, peptides and/or steroids. The amount of each additional component added will depend on the particular nature of the component and the purpose for adding the component. Typically, however, the amount of each optional component will be below about 10% by weight of the total weight of the blend.

The phrase "total weight of the blend" is intended to include the weight of GTMC and poly(oxyalkylene) and any additional ingredients included in the blend, but is intended to exclude the weight of the solvent, if any, employed in mixing or applying the blend.

The preparation and application of the polymer blends of the present invention may be accomplished by any suitable method which provides a substantially homogenous mixture of the two principal polymer components (i.e., GTMC and poly(oxyalkylene)). For example, the bioabsorbable copolymer and poly(oxyalkylene) may be melt blended. Preferably, the components of the blends are dissolved in a solvent and mixed to provide a substantially homogenous solution. The solution is applied to a substrate such as a medical device, and the solvent is removed, such as for example, by evaporation.

More particularly, in forming the solution it is preferred to first dissolve the GTMC polymer in just enough solvent to dissolve the polymer with stirring. Then the poly(oxyalkylene) is slowly added with continued stirring. If necessary, additional amounts of solvent may be added to effectuate complete dissolving of the poly(oxyalkylene).

The compositions of this invention form absorbable films having good durability, elastic and hydrophilic or hydrophobic properties. They may be used for surface coating and/or encapsulating and may act to fill voids or interstices in medical devices, such as, for example, vascular graft materials or beads used to form orthopedic or dental implants. The coating compositions may be applied to absorbable or non-absorbable substrates.

### EXAMPLE

### Copolymer Preparation

A copolymer of glycolide and trimethylene carbonate was prepared by melt phase ring opening copolymerization of 28.42 grams (35 mole percent) glycolide and 46.41 grams (65 mole percent) trimethylene carbonate in the presence of stannous octoate (1.9 cc of .2 gms of stannous octoate dissolved in 25 cc of ether) in an appropriate reaction vessel at 160°C for 12 hours. After polymerization the vessel was allowed to cool to room temperature. The polymer was then removed from the vessel, dried in vacuum and kept dry until used.

### Polymer Blend Preparation

22.5 grams of dried poly (glycolide-co-trimethylene carbonate) was placed into a round bottom flask and dissolved in about 200 ml of methylene chloride. Polyethylene oxide (2.5 gms; molecular weight 8000) was slowly added to this solution while stirring. After complete addition of polyethylene oxide, stirring of the mixture was continued for 16 hours. After thorough mixing, the solution mixture was poured into a dish and the excess solvent was evaporated. The thick film formed was dried well under vacuum (24 hours at room temperature) and stored dry. The resulting material contained 90% GTMC copolymer and 10% PEO.

### Coating Onto Vascular Graft Tubes

A polymer blend was prepared as described above containing 20 percent polyethylene oxide based on the total weight of the blend. A 5% solution of the blend in methylene chloride was applied to a length of a vascular graft tube having a diameter of 2-3 mm by dipping the tube into the solution and evaporating off the solvent. The vascular graft tube was woven from filaments of both absorbable and non-absorbable materials; namely, filaments of poly(glycolide/lactide) and filaments of Hytrel 5556 (an elastomer commercially available from E. I. duPont de Nemours & Co., Delaware). In vitro testing suggests that the blend of this Example would begin to absorb in about 2-3 hours after implantation into a mammalian body.

It is not intended to limit the present invention to the specific embodiments described above. It is recognized that changes may be made in the coating compositions specifically described herein without departing from the scope and teaching of the instant invention, and it is intended to encompass all other embodiments, alternatives and modifications consistent with the invention.

## Claims

1. A bioabsorble composition comprising a mixture of:
a bioabsorbable copolymer having soft segments; and
a poly(oxyalkylene).

2. A composition according to claim 1 wherein said soft segments are provided in said copolymer by one or more comonomers selected from trimethylene carbonate, dioxanone, coprolactones, alkylene oxylates, hydroxybutyrates, esteramides, hydroxyvalerates, urethanes, hexamethylene carbonate, and mixtures thereof.

3. A composition according to claim 1 or 2 wherein said soft segment comonomer is present in said copolymer in an amount of up to about 75 mole %.

4. A composition according to claim 3 wherein said soft segment comonomer is present in said copolymer in an amount of about 65 %.

5. A composition according to any one of the preceding claims wherein said poly(oxyalkylene) is present in the mixture in an amount less than about 30 % based on the weight of the total mixture.

6. A composition according to claim 2 wherein said copolymer comprises one or more monomers selected from glycolide and lactide.

7. A composition according to any one of the preceding claims and including a medico-surgically useful substance which is an antithrombogenic agent.

8. A bioabsorbable composition comprising a mixture of:
a copolymer of glycolide and trimethylene carbonate; and
a poly(oxyalkylene).

9. A composition according to claim 8 wherein the copolymer comprises:
glycolide in an amount up to about 50 mole percent; and
trimethylene carbonate in an amount up to about 75 mole percent.

10. A composition according to claim 9 wherein said copolymer comprises about 35 mole percent glycolide and about 65 percent glycolide and about 65 percent mole trimethylene carbonate.

11. A composition according to any one of the preceding claims wherein said poly(oxyalkylene) is present in an amount from about 10 percent to about 20 percent based on the weight of the mixture.

12. A composition according to any one of the preceding claims wherein said poly(oxyalkylene) is selected from polyethylene oxide, polypropylene oxide, poly(oxyethylene-oxypropylene) copolymers, polypentylene oxide, poly-1,4-butane diol and block copolymers of polyoxyethylene and polyoxypropylene.

13. A method of coating a medical device comprising applying to the device a blend of a poly(oxyalkylene) and a bioabsorbable copolymer having soft segments.

14. A method as in claim 13 wherein said soft segments are provided in said copolymer by polymerizing one or more comonomers selected from trimethylene carbonate, dioxanone, caprolactone, alkylene oxalates, hydroxybutyrates, esteramides, hydroxyvalerates, urethanes, hexamethylene carbonate and mixtures thereof with one or more monomers selected from the group consisting of glycolide and lactide.

15. A method according to claim 13 or 14 wherein the step of applying said blend comprises:
preparing a solution by dissolving a poly(oxyalkylene) and a said copolymer in a solvent;
applying said solution to the medical device; and
removing said solvent.

16. A method as in claims 13, 14 or 15 wherein said blend further comprises an effective amount of a medico-surgically useful substance.

17. A method of coating a medical device comprising applying a blend of poly(oxyalkylene) and a copolymer of glycolide and trimethylene carbonate to at least a portion of the medical device.
